# EUROPEAN PATENT APPLICATION

(11) **EP 4 817 534 A1**
(43) Date of publication of application: **07.10.2026**
(21) Application number: 23960117.2
(22) Date of filing: 28.11.2023
(51) Int. Cl.: A61B 17/221

(54) **CRUSHING TOOL AND ATTACHMENT FOR CRUSHING TOOL**

(71) Applicant: Olympus Medical Systems Corp., Tokyo 192-8507 (JP); National University Corporation Kobe University, Kobe-shi, Hyogo 657-8501 (JP)
(72) Inventor: SAKAI, Arata, Kobe-shi, Hyogo 657-8501 (JP); KODAMA, Yuzo, Kobe-shi, Hyogo 657-8501 (JP); MASUDA, Atsuhiro, Kobe-shi, Hyogo 657-8501 (JP); KOBAYASHI, Takashi, Kobe-shi, Hyogo 657-8501 (JP); HATSUDA, Tomoki, Hachioji-shi, Tokyo 192-8507 (JP); YUASA, Masaru, Hachioji-shi, Tokyo 192-8507 (JP); SHOJI, Keita, Hachioji-shi, Tokyo 192-8507 (JP)
(74) Representative: Schicker, Silvia
(86) International application number: PCT/JP2023/042544
(87) International publication number: WO 2025/115096

(57) **Abstract**

A crushing tool includes a sheath, a distal end member attached to a distal end of the sheath, and a basket wire that protrudes from a distal end of the distal end member, wherein the distal end member has a wire guide that guides the basket wire, and the wire guide is disposed outward in a radial direction with respect to a distal end surface of the distal end member.

## Description

### TECHNICAL FIELD

The present disclosure relates to a crushing tool and an attachment for a crushing tool.

### BACKGROUND ART

Basket forceps (basket grasping forceps, basket-type grasping forceps) are used as crushing tools for crushing stones formed in a bile duct or the like. The basket forceps are formed in a basket shape using a wire. A user captures the stone in the basket forceps and closes the basket forceps to clamp the stone, thereby crushing it. Stone crushing using the basket forceps is simple and inexpensive. Meanwhile, it is often not possible to crush stones due to an insufficient crushing force.

Basket forceps with an improved crushing force have been devised to crush stones appropriately. For example, Patent Documents 1 and 2 disclose basket forceps that crush stones using a crushing member other than a wire.

### Citation List

### Patent Document

Patent Document 1: United States Patent No. 5176688
Patent Document 2: United States Patent No. 9168099

### SUMMARY OF INVENTION

### Technical Problem

However, the conventional basket forceps described in Patent Documents 1 and 2 may not be able to crush a captured stone according to a position of the stone. For example, when a stone is captured at a position that is deviated from a central axis of the basket forceps, it is difficult to apply a crushing force evenly to the stone, and thus it is difficult to crush the stone.

In consideration of the above circumstances, an object of the present disclosure is to provide a crushing tool and an attachment for a crushing tool that can crush stones appropriately regardless of a position of the stones that have been captured.

### Solution to Problem

To solve the above problems, this disclosure proposes the following means.

A crushing tool according to a first aspect of the present disclosure includes a sheath, a distal end member attached to a distal end of the sheath, and a basket wire that protrudes from a distal end of the distal end member, wherein the distal end member has a wire guide that guides the basket wire, and the wire guide is disposed outward in a radial direction with respect to a distal end surface of the distal end member.

An attachment for a crushing tool according to a second aspect of the present disclosure has a guide lumen through which a basket wire passes, and the guide lumen has an inclined surface that inclines outward in a radial direction from a proximal end to a distal end.

A crushing tool according to a third aspect of the present disclosure includes a sheath, a distal end member attached to a distal end of the sheath, and a basket formed by a basket wire protruding from a distal end of the distal end member, wherein when the basket wire is further pulled toward a proximal end side of the sheath in a state in which a stone is captured by the basket, the distal end of the distal end member is pressed against the stone.

### Advantageous Effects of Invention

According to the crushing tool and the attachment for a crushing tool of the present disclosure, stones can be crushed appropriately regardless of the position of the stone that has been captured.

### BRIEF DESCRIPTION OF DRAWINGS

[FIG. 1] An overall view showing an endoscopic treatment system according to a first embodiment.
[FIG. 2] An overall view showing a crushing tool of the endoscopic treatment system.
[FIG. 3] A perspective view showing a distal end portion of the crushing tool.
[FIG. 4] A cross-sectional view showing the distal end portion of the crushing tool.
[FIG. 5] A diagram showing a bile duct to be treated.
[FIG. 6] A diagram showing a capturing process.
[FIG. 7] A cross-sectional view showing the distal end portion of the crushing tool in a crushing step.
[FIG. 8] A cross-sectional view showing the distal end portion of the crushing tool in the crushing step.
[FIG. 9] A cross-sectional view showing the distal end portion of the crushing tool in the crushing step.
[FIG. 10] A diagram showing a conventional distal end member (a comparison example) that does not have a tapered portion.
[FIG. 11] A perspective view of a distal end portion of the crushing tool according to a modified example.
[FIG. 12] A perspective view of a distal end portion of a crushing tool according to a second embodiment.
[FIG. 13] A cross-sectional view showing the distal end portion of the crushing tool.
[FIG. 14] A front view showing the distal end portion of the crushing tool when seen from a distal end side.
[FIG. 15] A front view showing a modified example of the distal end portion of the crushing tool.
[FIG. 16] A perspective view showing a distal end portion of a crushing tool according to a third embodiment.
[FIG. 17] A cross-sectional view showing the distal end portion of the crushing tool.
[FIG. 18] A perspective view showing a distal end portion of the crushing tool according to a modified example.
[FIG. 19] A cross-sectional view showing the distal end portion according to the modified example.
[FIG. 20] A perspective view showing the distal end portion of the crushing tool according to the third embodiment.
[FIG. 21] A cross-sectional view showing the distal end portion of the crushing tool.
[FIG. 22] A perspective view of an attachment according to a fifth embodiment.
[FIG. 23] A cross-sectional view of the attachment.
[FIG. 24] A perspective view of a conventional crushing tool.
[FIG. 25] A perspective view of the attachment attached to the conventional crushing tool.

### DESCRIPTION OF EMBODIMENTS

### (First embodiment)

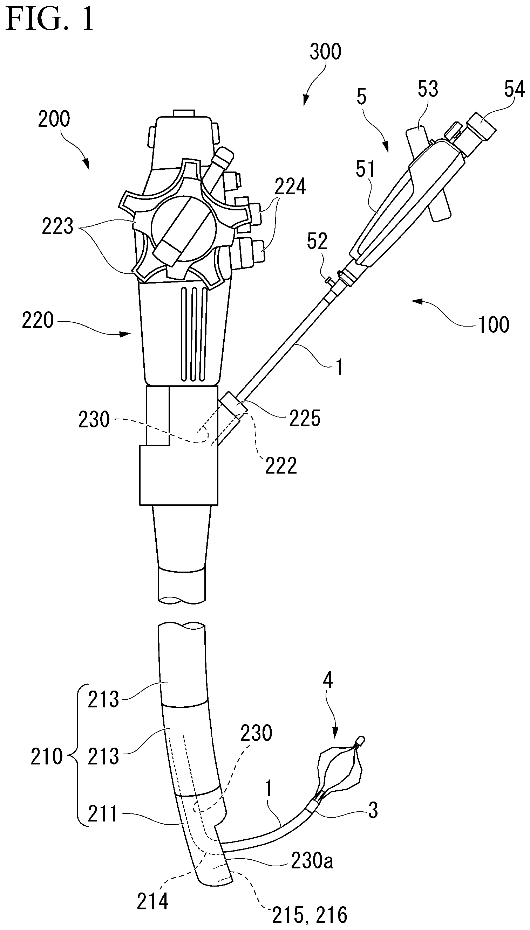

An endoscopic treatment system 300 including a crushing tool 100 according to a first embodiment of the present invention will be described with reference to FIGS. 1 to 10.

### [Endoscopic Treatment System 300]

FIG. 1 is an overall view of the endoscopic treatment system 300.

As shown in FIG. 1, the endoscopic treatment system 300 includes a crushing tool 100 and an endoscope 200. The crushing tool 100 is inserted into the endoscope 200 for use.

### [Endoscope 200]

The endoscope 200 is a known side-viewing flexible endoscope, and includes a long insertion part 210 and an operation part 220 provided at a proximal end portion of the insertion part 210. The endoscope 200 may also be a direct-viewing flexible endoscope.

The insertion part 210 has a distal end rigid part 211 provided at a distal end portion thereof, a bending part 212 that is provided on a proximal end side of the distal end rigid part 211 so as to be bent, and a flexible tube part 213 provided on a proximal end side of the bending part 212. An imaging unit 216 having a light guide 215 and a CCD is provided on a side surface of the distal end rigid part 211 in an exposed state.

The insertion part 210 is formed with a treatment tool channel 230 for inserting the crushing tool 100 therethrough. A distal end portion 230a of the treatment tool channel 230 opens at the side surface of the distal end rigid part 211. A proximal end portion of the treatment tool channel 230 extends to the operation part 220.

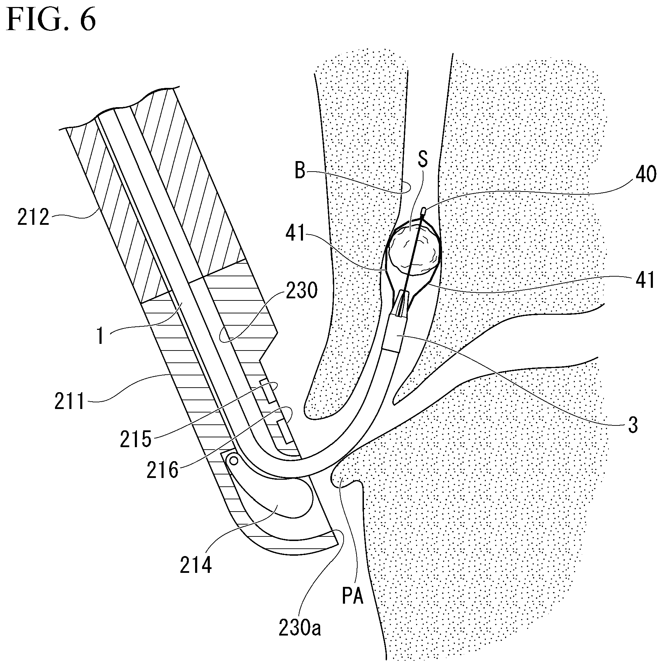

The distal end rigid part 211 of the treatment tool channel 230 is provided with a standing table 214 (refer to FIG. 6). A proximal end portion of the standing table 214 is rotatably supported by the distal end rigid part 211. A standing table operation wire (not shown) fixed to a distal end portion of the standing table 214 passes through the insertion part 210 and extends to the proximal end side.

The bending part 212 is configured to be bendable in an up-down direction and a left-right direction. A distal end of an operation wire is fixed to a distal end side of the bending part 212. The operation wire passes through the insertion part 210 and extends to the operation part 220.

A knob 223 for operating the operation wire and a switch 224 for operating the imaging unit 216, or the like are provided on a proximal end side of the operation part 220. A user can bend the bending part 212 in a desired direction by operating the knob 223.

A forceps port 222 that communicates with the treatment tool channel 230 is provided on the distal end side of the operation part 220. The user can insert the crushing tool 100 through the forceps port 222. A forceps plug 225 is attached to the forceps port 222 to prevent leakage of body fluids.

### [Crushing Tool 100]

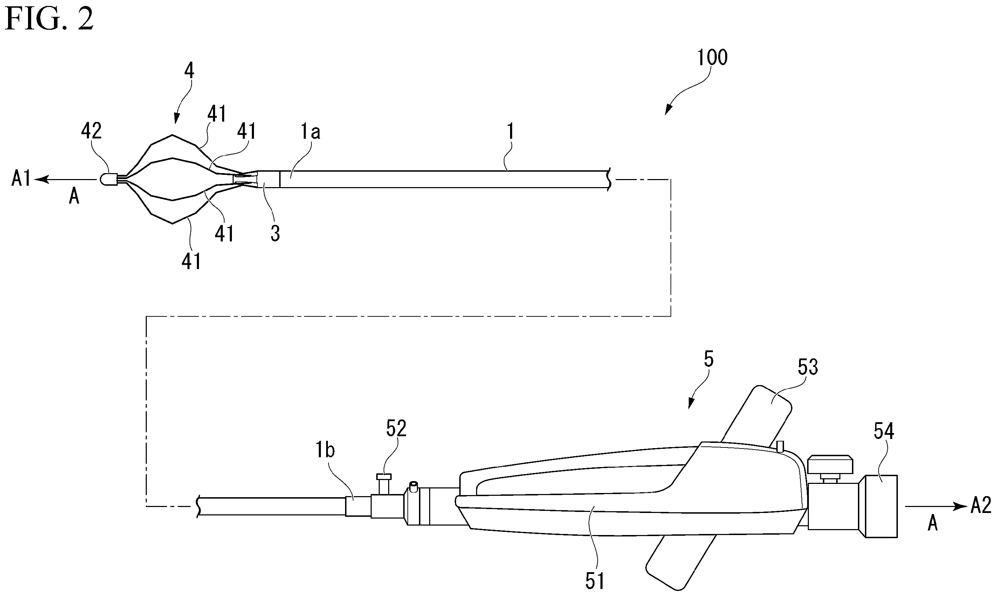

FIG. 2 is an overall view of the crushing tool 100.

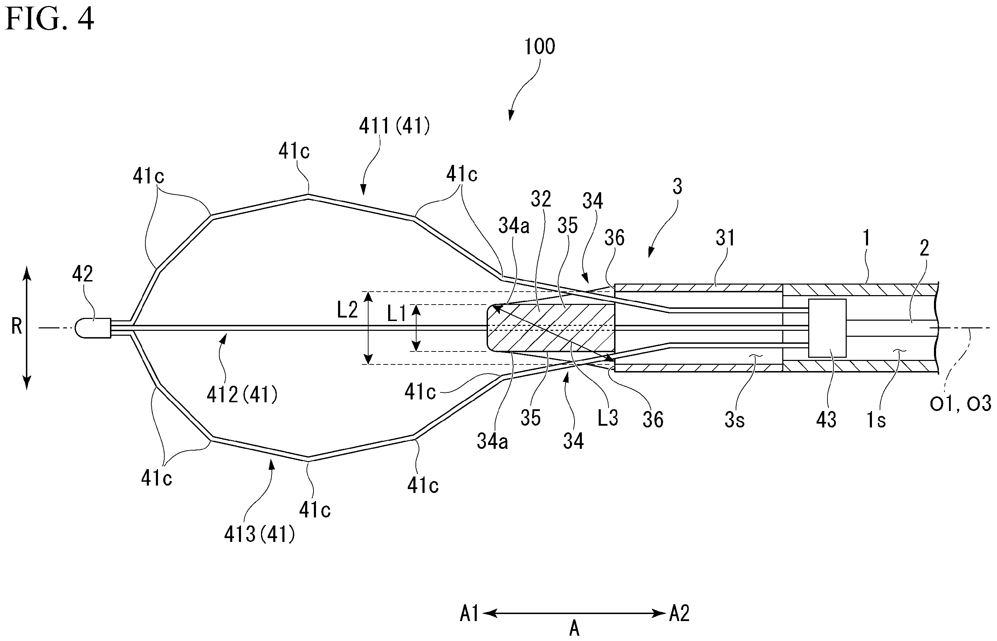

The crushing tool 100 (also referred to as an endoscopic treatment tool 100) includes a sheath 1, an operation wire 2 (refer to FIG. 4), a distal end member 3, a basket 4, and an operation part 5. In the following description, in a longitudinal direction A of the crushing tool 100, a side that is inserted into a patient's body will be referred to as a "distal end side (a distal side) A1", and a side of the operation part 5 will be referred to as a "proximal end side (a proximal side) A2".

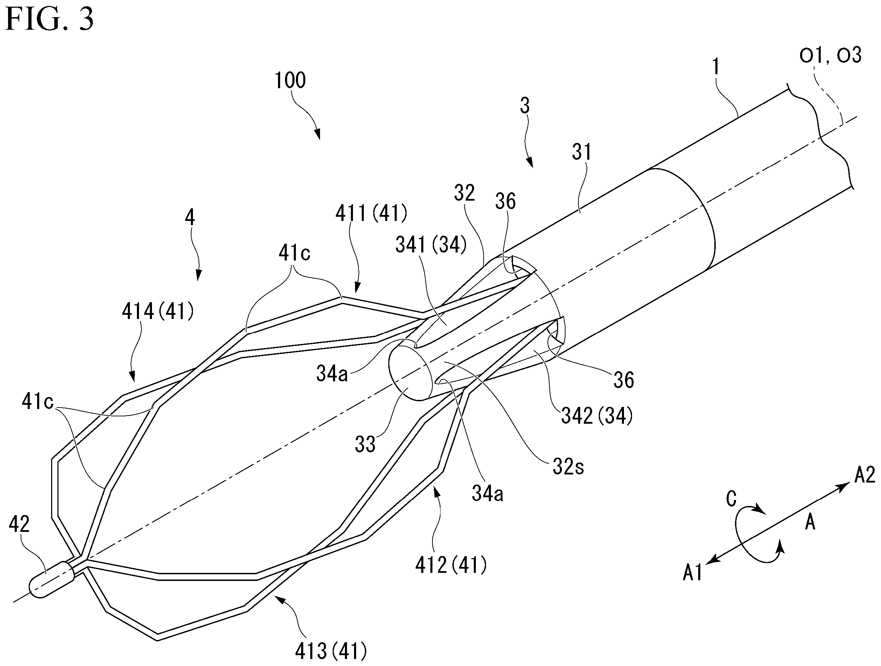

FIG. 3 is a perspective view of the distal end portion of the crushing tool 100.

The sheath 1 is a flexible, elongated member that extends from a distal end 1a to a proximal end 1b. The sheath 1 has an outer diameter that allows it to be inserted into the treatment tool channel 230 of the endoscope 200. As shown in FIG. 1, in a state in which the sheath 1 is inserted into the treatment tool channel 230, the distal end 1a of the sheath 1 can protrude and retract from a distal end opening 230a of the treatment tool channel 230. The operation wire 2 is inserted through an internal space (a lumen) 1s of the sheath 1.

The operation wire 2 is a metal wire that is inserted through the internal space 1s of the sheath 1. A distal end of the operation wire 2 is connected to the basket 4, and a proximal end of the operation wire 2 is connected to the operation part 5.

FIG. 4 is a cross-sectional view of the distal end portion of the crushing tool 100.

The distal end member 3 is attached to the distal end 1a of the sheath 1. The distal end member 3 is made of a metal such as stainless steel. A central axis O3 of the distal end member 3 in the longitudinal direction A coincides with a central axis O1 of the sheath 1 in the longitudinal direction A. The distal end member 3 has a main body 31, a tapered portion 32, and a wire guide 34.

The main body 31 is formed in a cylindrical shape and is attached to the distal end 1a of the sheath 1. An internal space 3s of the main body 31 communicates with the internal space 1s of the sheath.

The tapered portion 32 is formed in a conical shape, and a length in a radial direction R is short from the proximal end side A2 to the distal side A1. A flat distal end surface 33 is formed at a distal end of the tapered portion 32. The distal end surface 33 is a plane perpendicular to the longitudinal direction A of the crushing tool 100. The tapered portion 32 is formed with four wire guides 34 in a circumferential direction C relative to the longitudinal direction A. The distal end surface 33 does not necessarily have to be a plane perpendicular to the longitudinal direction A of the crushing tool 100, but may be, for example, a curved surface that is convex toward the distal side A1.

The wire guides 34 are grooves that extend in the longitudinal direction A on the outer circumference of the tapered portion 32. The four wire guides 34 are disposed at different positions in the circumferential direction C of the tapered portion 32. The four wire guides 34 are disposed outward in the radial direction R with respect to the central axis O3. The four wire guides 34 are separated by walls 35 provided inward in the radial direction R of the distal end member 3.

The wire guide 34 includes a first wire guide 341, a second wire guide 342, a third wire guide 343, and a fourth wire guide 344. The first wire guide 341, the second wire guide 342, the third wire guide 343, and the fourth wire guide 344 are disposed at equal intervals in the circumferential direction C.

The first wire guide 341 and the third wire guide 343 are disposed on both sides of the central axis O3. The second wire guide 342 and the fourth wire guide 344 are disposed on both sides of the central axis O3.

A distal end 34a of the wire guide 34 is located on a side surface 32s of the tapered portion 32. Therefore, the wire guide 34 does not extend to the distal end surface 33. In other words, the distal end surface 33 is located closer to a distal tip 42 side (the distal side A1) than the distal end 34a of the wire guide 34.

A proximal end 34b of the wire guide 34 extends to the main body 31. An opening (a wire insertion part, a guide lumen) 36 that communicates with the internal space 3s of the main body 31 is formed between the main body 31 and the wire guide 34. The distal end surface 33 is located closer to the distal tip 42 side (the distal side A1) than the opening 36.

A length L1 of the distal end surface 33 of the distal end member 3 in the radial direction R is preferably smaller than an inner diameter L2 at a distal end of the main body 31. Furthermore, a maximum distance L3 from the opening 36 to the distal end surface 33 is preferably greater than the inner diameter L2 of the main body 31.

The basket 4 is formed in a basket shape by four basket wires 41. The distal ends of the four basket wires 41 are attached to the distal tip 42. The proximal ends of the four basket wires 41 are attached to a bundled portion 43 provided at the distal end of the operation wire 2. The basket wires 41 are made of a single wire or a stranded wire made of a highly elastic material. The basket wires 41 may be made of, for example, a nickel-titanium alloy, stainless steel, a stainless steel alloy, or the like.

The basket wires 41 protrude from the internal space 3s of the main body 31 to the distal side A1 through the opening 36. The basket wires 41 protruding from the opening 36 to the distal side A1 are guided in advance and retraction directions by the wire guide 34.

As shown in FIG. 4, the basket wires 41 have a plurality of curved portions 41c formed between the distal tip 42 and the bundled portion 43. Each of the basket wires 41 does not necessarily have to have the plurality of curved portions 41c, and may be smoothly curved from the distal tip 42 to the bundled portion 43. The basket 4 is configured to expand into a basket shape in a natural state by the plurality of basket wires 41.

The basket wires 41 include a first basket wire 411, a second basket wire 412, a third basket wire 413, and a fourth basket wire 414. The first basket wire 411, the second basket wire 412, the third basket wire 413, and the fourth basket wire 414 are disposed at equal intervals in the circumferential direction C.

The first basket wires 411 and the third basket wires 413 are disposed on both sides of the central axis O3. The second basket wires 412 and the fourth basket wires 414 are disposed on both sides of the central axis O3.

The first basket wires 411 are guided by the first wire guide 341. The second basket wires 412 are guided by the second wire guide 342. The third basket wire 413 is guided by the third wire guide 343. The fourth basket wire 414 is guided by the fourth wire guide 344.

The number of basket wires 41 is not limited to four, and may be, for example, three, five, or six. The number of wire guides 34 is changed according to the number of basket wires 41.

The operation part 5 is provided on the proximal end side A2 of the sheath 1. The operation part 5 includes an operation part main body 51, a slider 53, and a guide wire port 54.

A distal end portion of the operation part main body 51 is connected to the proximal end 1b of the sheath 1. The operation wire 2 extends from the proximal end 1b of the sheath 1. The operation wire 2 is fixed to the slider 53.

A liquid supply port 52 is provided in the operation part main body 51. The liquid supply port 52 is connected to the proximal end of the sheath 1 via a conduit formed in the operation part main body 51. A liquid supplied from the liquid supply port 52 passes through the sheath 1 and is discharged from the distal end member 3.

The slider 53 is attached to the operation part main body 51 to be movable in the longitudinal direction A. The proximal end of the operation wire 2 is fixed to the slider 53. When the user advances and retracts the slider 53 relative to the operation part main body 51, the operation wire 2 advances and retracts.

The guide wire port 54 is an opening through which a guide wire can be inserted.

### [Operation of Endoscopic Treatment System 300]

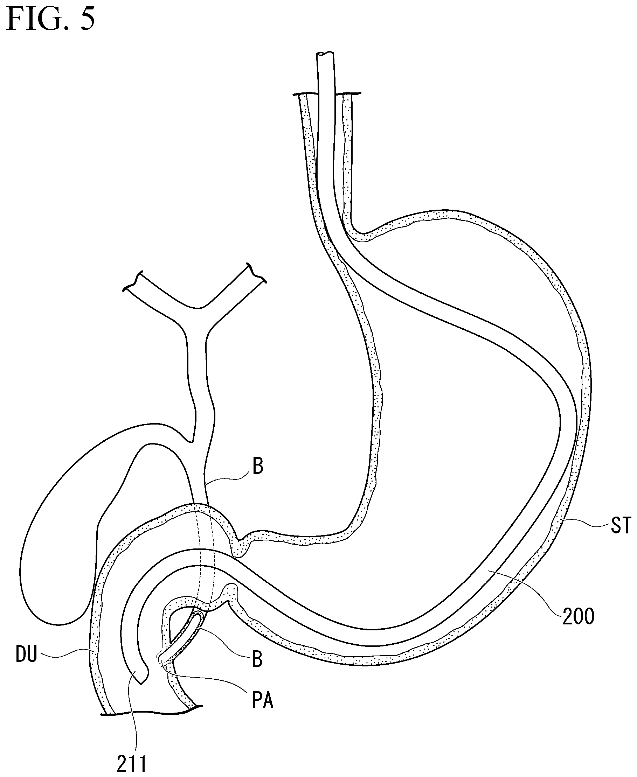

Next, an Operation of the endoscopic treatment system 300 according to the embodiment will be described. Specifically, a procedure for crushing a stone S formed in a bile duct B will be described. FIG. 5 shows the bile duct B as a treatment target.

### <Step S1: Endoscope insertion process>

A surgeon inserts the insertion part 210 of the endoscope 200 into a patient's lumen through a natural orifice such as a mouth. The surgeon operates the operation part 220 as necessary to bend the bending part 212. The surgeon inserts the distal end rigid part 211 of the endoscope 200 into a duodenum DU. The surgeon arranges the distal end rigid part 211 at a position at which it faces an opening PA1 of a papilla PA.

### <Step S2: Insertion process>

The surgeon inserts the crushing tool 100 into the bile duct B through the opening PA1 of the papilla PA. Specifically, the crushing tool 100 is inserted into the treatment tool channel 230 of the endoscope 200, and the basket 4 of the crushing tool 100 protrudes from the distal end portion 230a, and in a state in which the basket 4 is closed, the basket 4 of the crushing tool 100 is inserted into the bile duct B through the opening of the papilla PA. The surgeon may place the guide wire in the bile duct B and insert the basket 4 of the crushing tool 100 into the bile duct B along the guide wire.

### <Step S3: Capturing process>

FIG. 6 shows a capturing process.

The surgeon opens the basket 4 and captures the stone S into the basket 4. Specifically, the stone S is taken into the basket 4 through the basket wires 41 and captured. At this time, the stone S is located away from the distal end surface 33, and the basket 4 holds the stone S with a force that does not crush the stone S.

### <Step S4: Crushing process>

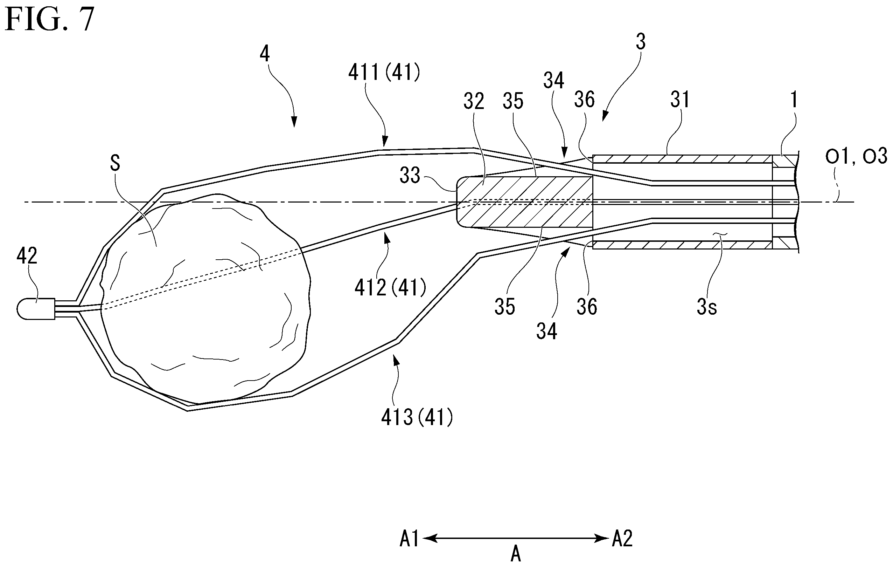

FIGS. 7 to 9 are cross-sectional views of the distal end portion of the crushing tool 100 during a crushing process.

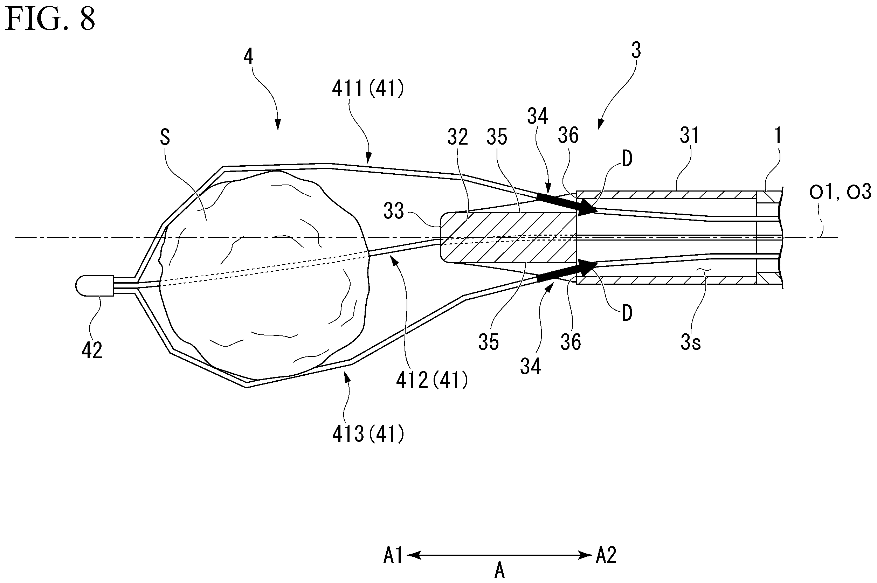

As shown in FIG. 7, it is assumed that the stone S is captured at a position offset from the central axis O3 and on the third basket wire 413 side with respect to the central axis O3. As shown in FIG. 8, the surgeon operates the slider 53 of the operation part 5 to retract the operation wire 2, thereby drawing the basket 4 toward the proximal end side A2. Since the wire guides 34 guide the basket wires 41 being pulled, directions D in which the four basket wires 41 are pulled tend to be the same direction. As a result, as the four basket wires 41 are pulled in, the stone S approaches the central axis O3.

The four wire guides 34 are separated by the walls 35 provided on the inside of the distal end member 3 in the radial direction R. The walls 35 restrict the basket wires 41 from moving beyond the central axis O3 to the opposite side in the radial direction R. As a result, as the four basket wires 41 are pulled in, it is possible to restrictthe stone S from moving away from the central axis O3.

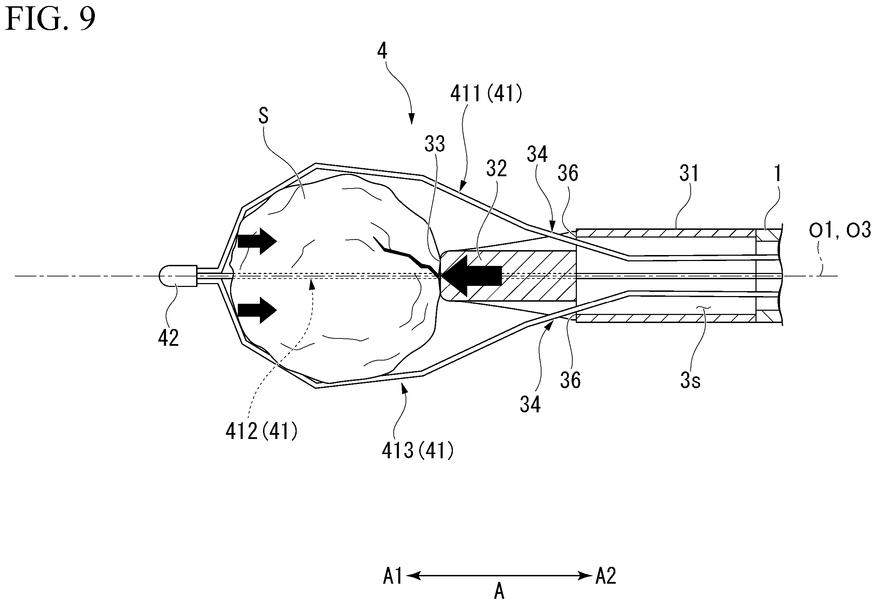

As shown in FIG. 9, in a state in which the stone S is captured by the basket 4, the surgeon further pulls the basket wire 41 toward the proximal end side A2. Since the stone S approaches the central axis O3, the stone S is firmly pressed against the distal end surface 33 of the distal end member 3. Therefore, the stone S is unlikely to slip off the distal end surface 33. Therefore, the surgeon can place the stone S between the basket wire 41 on the distal side A1 and the distal end surface 33 on the proximal end side A2, and crush the stone S with a small amount of force.

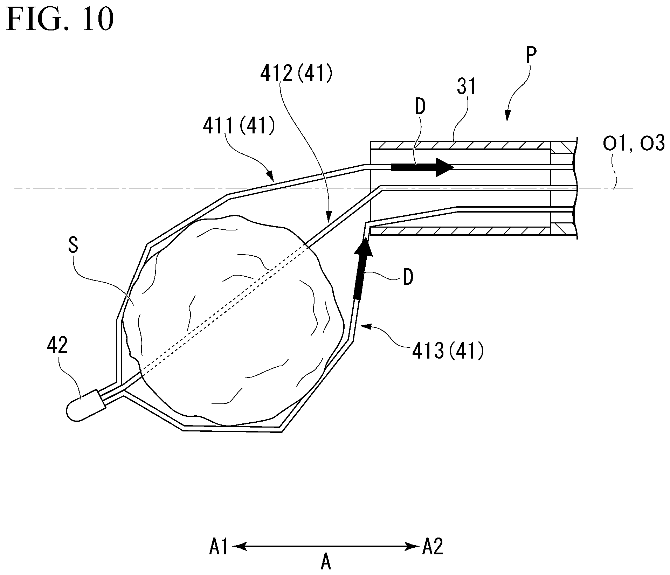

FIG. 10 is a diagram showing a conventional distal end member P (a comparison example) that does not have the tapered portion 32.

The conventional distal end member P has a cylindrical shape without the tapered portion 32 and the wire guide 34 (the wall 35). Therefore, when the basket 4 is pulled toward the proximal end side A2, the four basket wires 41 are unlikely to be pulled in the same direction D. Furthermore, the conventional distal end member P does not have the wall 35. Therefore, it is not possible to restrict the basket wires 41 from moving beyond the central axis O3 to the opposite side in the radial direction R. In FIG. 10, the first basket wire 411 has moved beyond the central axis O3 to the opposite side in the radial direction R.

According to the crushing tool 100 of the embodiment, the stone S can be crushed appropriately regardless of the position of the stone S captured into the basket 4.

The first embodiment of the present invention has been described above in detail with reference to the drawings, but the specific configuration is not limited to the embodiment, and design changes and the like are also included within the scope that does not deviate from the gist of the present invention. Furthermore, the components shown in the above-described embodiment and modified example can be appropriately combined to form a configuration.

### (Modified example 1)

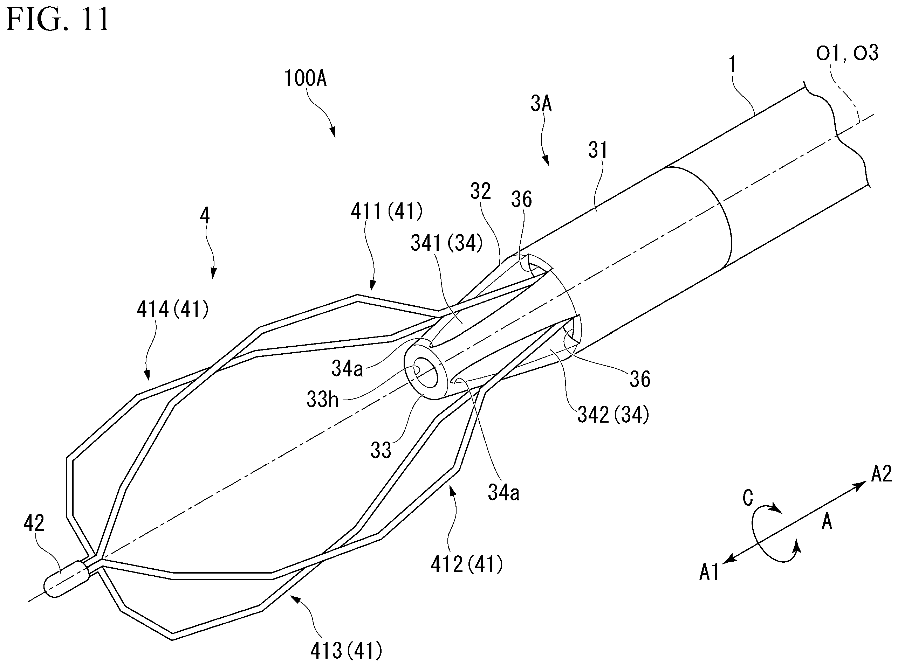

FIG. 11 is a perspective view of a distal end portion of a crushing tool 100A, which is a modified example of the crushing tool 100.

Compared to the crushing tool 100, the crushing tool 100A includes a distal end member 3A which is a modified example of the distal end member 3. An opening 33h is formed in the distal end surface 33 of the distal end member 3A. The opening 33h communicates with the internal space 3s of the main body 31. The opening is disposed inward in the radial direction R with respect to the distal end 34a of the wire guide 34. The opening 33h does not necessarily need to communicate with the internal space 3s of the main body 31, and may be a hole with a bottom provided from the distal end surface 33 toward the opening 36.

### (Second embodiment)

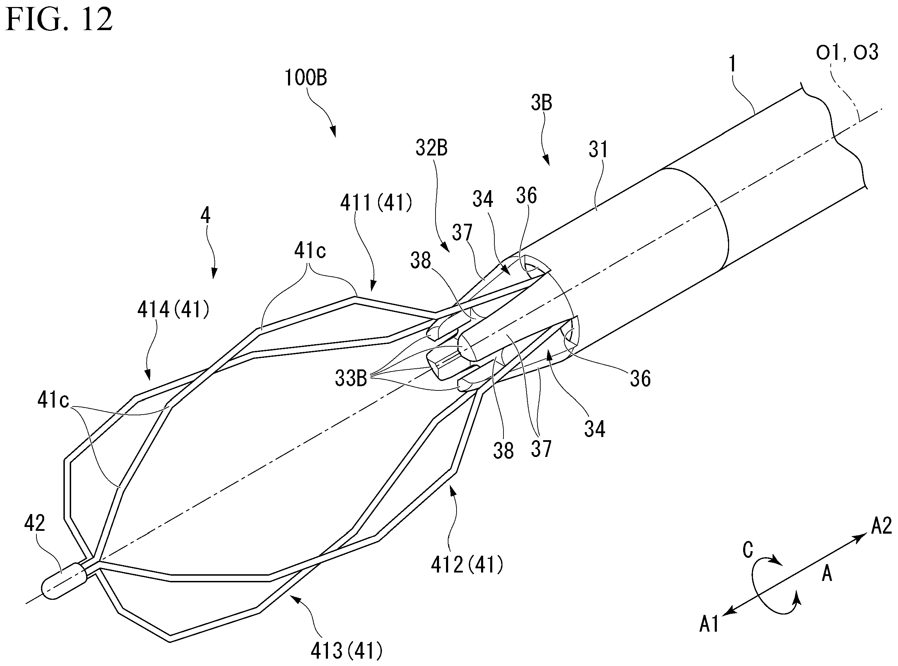

A crushing tool 100B according to a second embodiment of the present invention will be described with reference to FIGS. 12 to 14. In the following description, components common to those already described will be designated by the same reference numerals, and redundant description will be omitted.

FIG. 12 is a perspective view of a distal end portion of the crushing tool 100B.

Like the crushing tool 100 of the first embodiment, the crushing tool 100B is used together with an endoscope 200 as an endoscopic treatment system. The crushing tool 100B includes the sheath 1, the operation wire 2, the distal end member 3B, the basket 4, and the operation part 5.

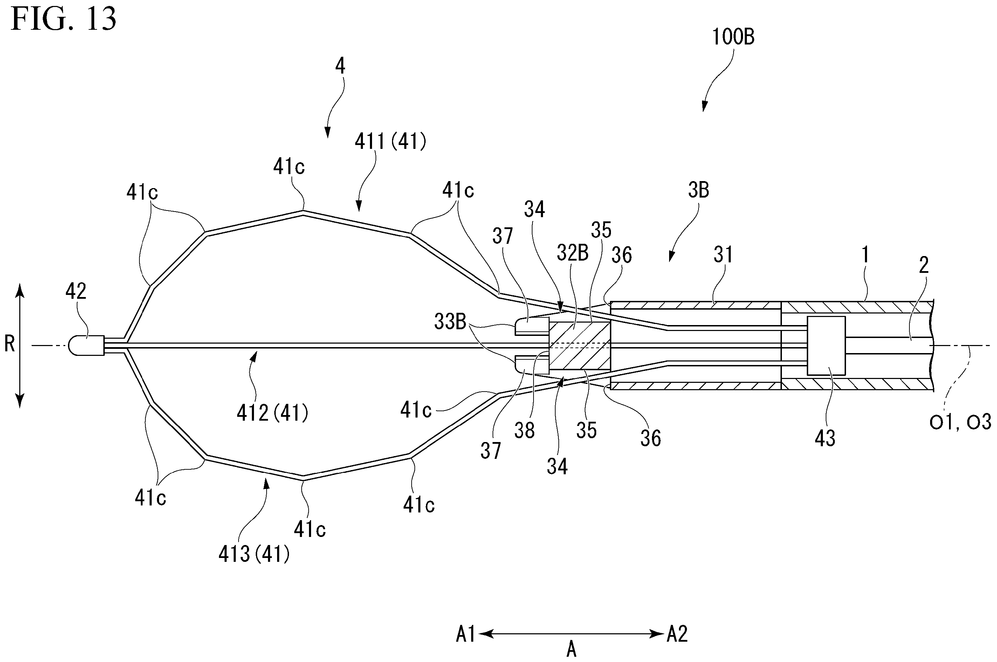

FIG. 13 is a cross-sectional view of the distal end portion of the crushing tool 100B.

The distal end member 3B is attached to the distal end 1a of the sheath 1. The distal end member 3B is made of a metal such as stainless steel. A central axis O3 of the distal end member 3B in the longitudinal direction A coincides with the central axis O1 of the sheath 1 in the longitudinal direction A. The distal end member 3B has a main body 31, a distal end portion 32B, and four wire guides 34.

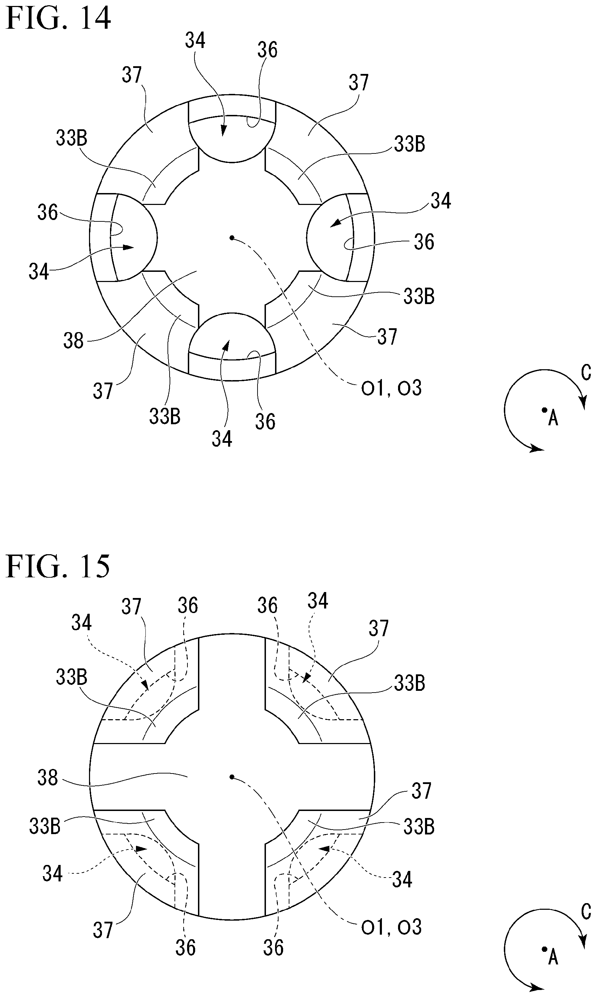

FIG. 14 is a front view of the distal end portion of the crushing tool 100B, seen from the distal side A1.

The distal end portion 32B has four protrusions 37. The wire guides 34 and the protrusions 37 are arranged alternately in the circumferential direction C. A curved or flat distal end surface 33B is formed at a distal end of each of the four protrusions 37. A slit 38 is formed in the distal end surface 33B.

The slit 38 passes through the central axis O3 of the distal end member 3 and extends in the radial direction R. The slit 38 is continuous with a distal end of the wire guide 34. As shown in FIG. 15, in a front view seen from the distal side A1 in the longitudinal direction A, the slits 38 and the distal ends of the wire guides 34 may be arranged alternately in the circumferential direction C.

According to the crushing tool 100B of the embodiment, since the slits 38 are formed in the distal end surface 33B, the stone S is less likely to slip off from the distal end surface 33B.

The second embodiment of the present invention has been described above in detail with reference to the drawings, but the specific configuration is not limited to the embodiment, and design changes and the like are also included within the scope that does not deviate from the gist of the present invention. Furthermore, the components shown in the above-described embodiment and modified example can be appropriately combined to form a configuration.

In the above embodiment, the number of the protrusions 37 having the distal end surface 33B is four, but the number of the protrusions 37 is not limited thereto. From the viewpoint of fixing the stone S, it is desirable that the number of the protrusions 37 be at least three or more.

### (Third embodiment)

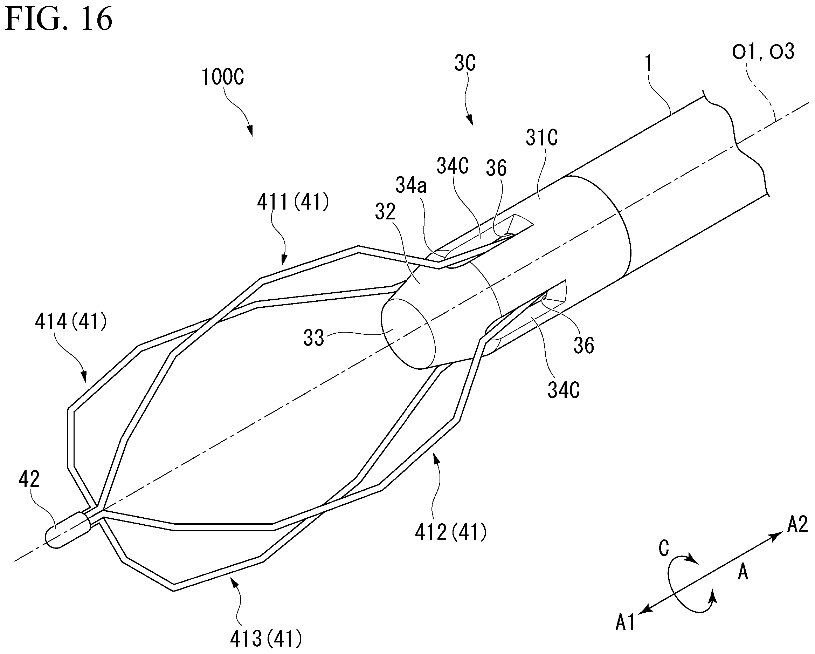

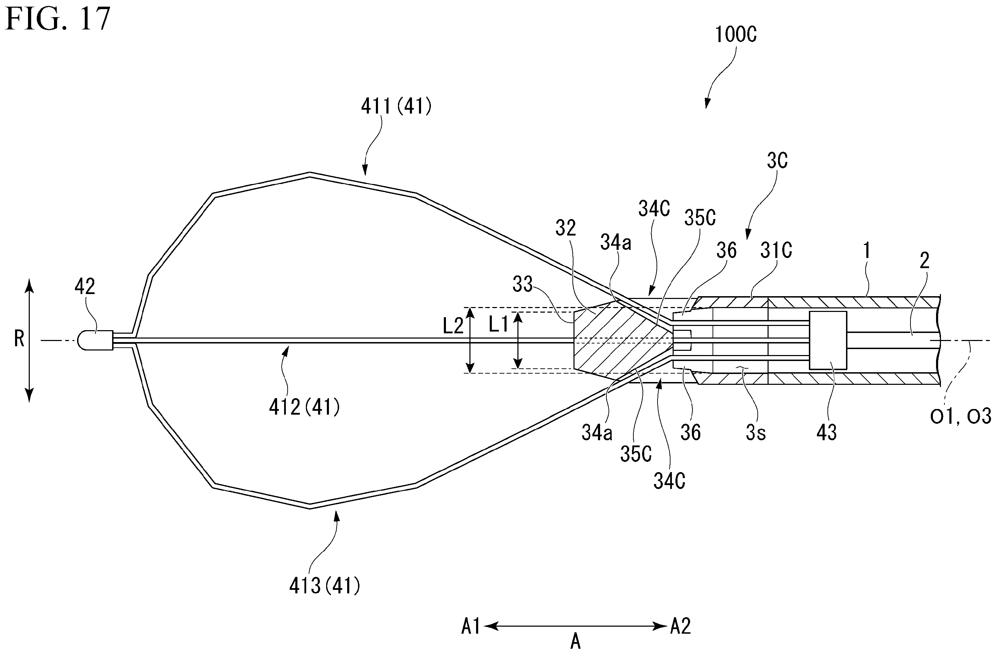

A crushing tool 100C according to a third embodiment of the present invention will be described with reference to FIGS. 16 and 17. In the following description, components common to those already described will be designated by the same reference numerals, and redundant description will be omitted.

FIG. 16 is a perspective view of a distal end portion of the crushing tool 100C.

Like the crushing tool 100 of the first embodiment, the crushing tool 100C is used together with an endoscope 200 as an endoscopic treatment system. The crushing tool 100C includes the sheath 1, the operation wire 2, a distal end member 3C, the basket 4, and the operation part 5.

FIG. 17 is a cross-sectional view of the distal end portion of the crushing tool 100C.

The distal end member 3C is attached to the distal end 1a of the sheath 1. The distal end member 3C is made of a metal such as stainless steel. A central axis O3 of the distal end member 3C in the longitudinal direction A coincides with the central axis O1 of the sheath 1 in the longitudinal direction A. The distal end member 3C has a main body 31C, a tapered portion 32, and a wire guide 34C.

The main body 31C is formed in a cylindrical shape and is attached to the distal end 1a of the sheath. An internal space 3s of the main body 31C communicates with the internal space 1s of the sheath 1. The main body 31C is formed with four wire guides 34C in the circumferential direction C relative to the longitudinal direction A.

The wire guides 34C are grooves that extend on the outer circumference of the main body 31C in the longitudinal direction A. The four wire guides 34C are disposed at different positions in the circumferential direction C of the main body 31C. The four wire guides 34C are disposed outward in the radial direction R with respect to the central axis O3. The four wire guides 34C are separated by inclined walls 35C provided inward in the radial direction R of the distal end member 3C.

The inclined wall (an inclined surface) 35C is a surface that inclines outward in the radial direction R as it goes from the proximal end to a distal end of the distal end member 3C. When the basket wires 41 advance or retreat, the basket wires 41 are guided by the inclined wall 35C.

A distal end 34a of the wire guide 34C is located on a side surface 32s of the tapered portion 32. Therefore, the wire guide 34C does not extend to the distal end surface 33. In other words, the distal end surface 33 is located closer to the distal tip 42 side (the distal side A1) than the distal end 34a of the wire guide 34C.

A proximal end 34b of the wire guide 34C extends to the main body 31C. An opening (a wire insertion part, a guide lumen) 36 that communicates with the internal space 3s of the main body 31 is formed in the main body 31C. The distal end surface 33 is located closer to the distal tip 42 side (the distal side A1) than the opening 36.

Preferably, the length L1 in the radial direction R of the distal end surface 33 of the distal end member 3C is smaller than the inner diameter L2 at the distal end of the main body 31C.

The number of basket wires 41 is not limited to four, and may be, for example, three, five, or six. The number of wire guides 34C is changed according to the number of basket wires 41.

According to the crushing tool 100C of the embodiment, since the wire guide 34C has the inclined wall 35C, the basket wire 41 can be easily inserted, and an opening and closing force of the basket 4 is reduced. In addition, a larger force is transmitted from the operation part 5 to the stone S. Furthermore, when an exposed position of the basket wire 41 is on an outer side in the radial direction R, the basket wire 41 can more easily follow the stone S when holding it. Therefore, a length of the tapered portion 32 in the longitudinal direction A can be shortened. As a result, the endoscope insertion performance is improved, and stability when the distal end surface 33 collides with the stone S is also improved.

The third embodiment of the present invention has been described above in detail with reference to the drawings, but the specific configuration is not limited to the embodiment, and design changes and the like are also included within the scope that does not deviate from the gist of the present invention. Furthermore, the components shown in the above-described embodiment and modified example can be appropriately combined to form a configuration.

### (Modified example)

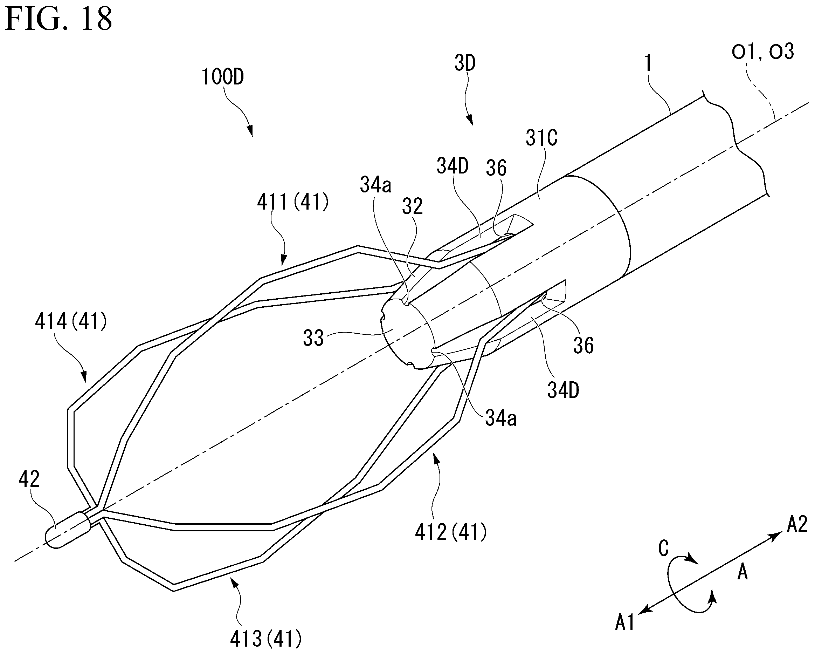

FIG. 18 is a perspective view of a distal end portion of a crushing tool 100D, which is a modified example of the crushing tool 100C. The crushing tool 100D includes the sheath 1, the operation wire 2, a distal end member 3D, the basket 4, and the operation part 5.

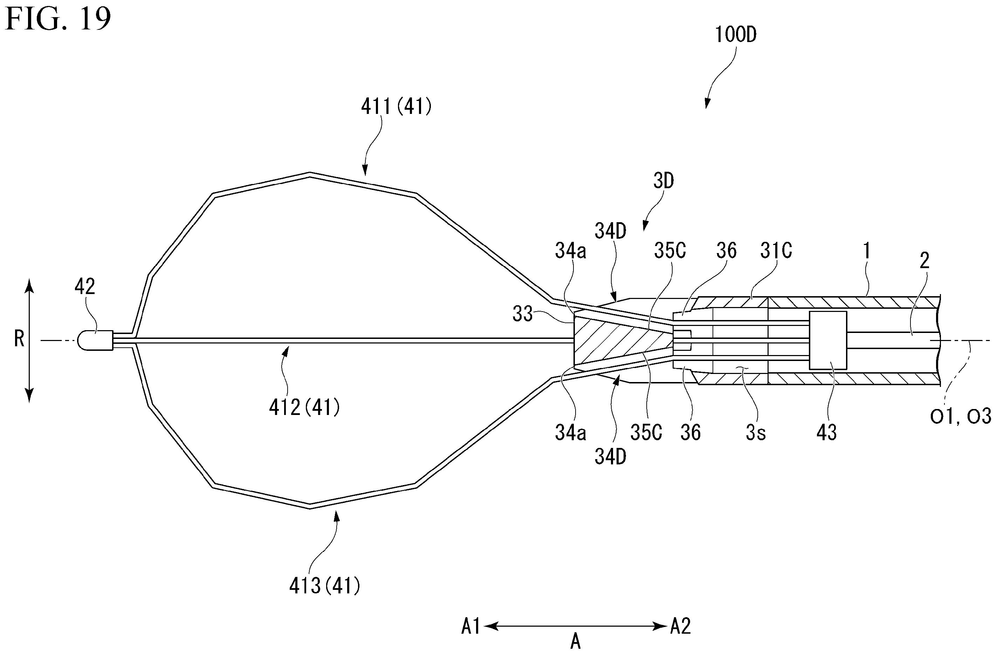

FIG. 19 is a cross-sectional view of the distal end portion of the crushing tool 100D.

Compared to distal end member 3C, the distal end member 3D has a wire guide 34D instead of the wire guide 34C. The distal end member 3D has a main body 31C, a tapered portion 32, and a wire guide 34D.

The wire guide 34D is a groove that extends in the longitudinal direction A on the outer circumferences of the main body 31C and the tapered portion 32. Four wire guides 34D are disposed at different positions in the circumferential direction C of the main body 31C. The four wire guides 34D are disposed outward in the radial direction R with respect to the central axis O3. The four wire guides 34D are separated by inclined walls 35C provided inward in the radial direction R of the distal end member 3C.

A distal end 34a of the wire guide 34D extends to the distal end surface 33 of the tapered portion 32. The distal end 34a of the wire guide 34D is disposed on a circumferential edge portion of the distal end surface 33.

### (Fourth embodiment)

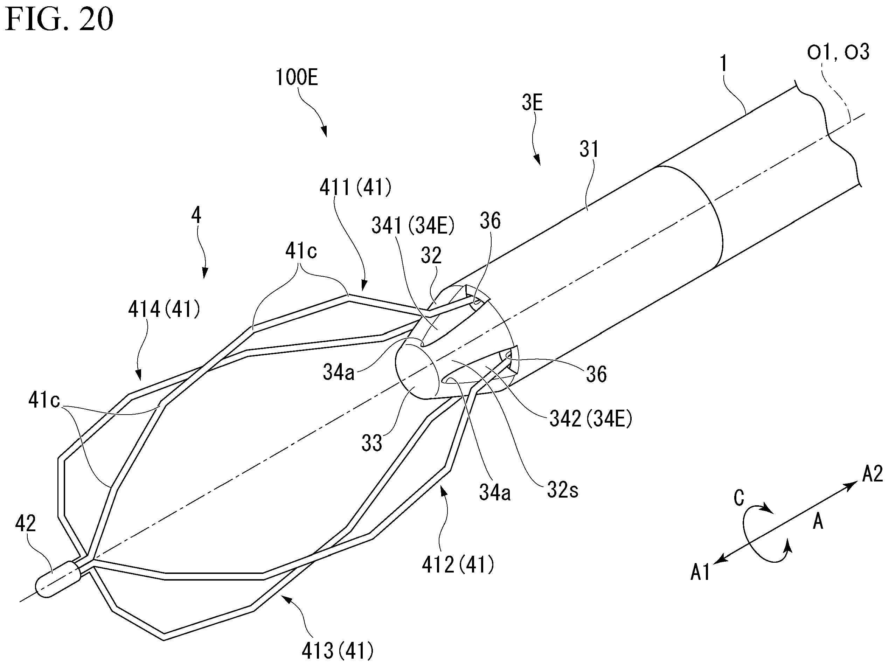

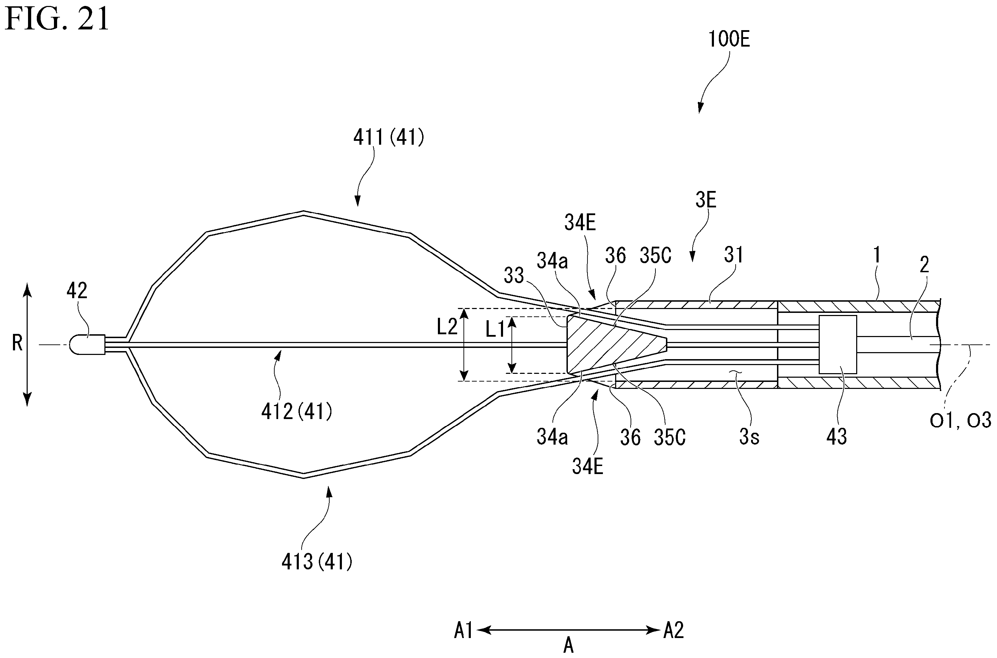

A crushing tool 100E according to a fourth embodiment of the present invention will be described with reference to FIGS. 20 and 21. In the following description, components common to those already described will be designated by the same reference numerals, and redundant description will be omitted.

FIG. 20 is a perspective view of a distal end portion of the crushing tool 100E.

Like the crushing tool 100 of the first embodiment, the crushing tool 100E is used together with an endoscope 200 as an endoscopic treatment system. The crushing tool 100E includes the sheath 1, the operation wire 2, a distal end member 3E, the basket 4, and the operation part 5.

FIG. 21 is a cross-sectional view of the distal end portion of the crushing tool 100E.

The distal end member 3E is attached to the distal end 1a of the sheath 1. The distal end member 3E is made of a metal such as stainless steel. A central axis O3 of the distal end member 3E in the longitudinal direction A coincides with the central axis O1 of the sheath 1 in the longitudinal direction A. The distal end member 3E has a main body 31, a tapered portion 32, and a wire guide 34E.

The wire guide 34E is a groove that extends in the longitudinal direction A on the outer circumference of the tapered portion 32. Four wire guides 34E are disposed at different positions in the circumferential direction C of the main body 31. The four wire guides 34E are disposed outward in the radial direction R with respect to the central axis O3. The four wire guides 34E are separated by inclined walls 35C provided inward in the radial direction R of the distal end member 3E.

A distal end 34a of the wire guide 34E is located on a side surface 32s of the tapered portion 32. Therefore, the wire guide 34E does not extend to the distal end surface 33. In other words, the distal end surface 33 is located closer to the distal tip 42 side (the distal side A1) than the distal end 34a of the wire guide 34E.

A proximal end 34b of the wire guide 34E extends to the main body 31. An opening (a wire insertion part, a guide lumen) 36 that communicates with the internal space 3s of the main body 31 is formed between the main body 31 and the wire guide 34E. The distal end surface 33 is located closer to the distal tip 42 side (the distal side A1) than the opening 36.

Preferably, the length L1 in the radial direction R of the distal end surface 33 of the distal end member 3E is smaller than the inner diameter L2 of the distal end of the main body 31.

The number of basket wires 41 is not limited to four, and may be, for example, three, five, or six. The number of wire guides 34E is changed according to the number of basket wires 41.

According to the crushing tool 100E of the embodiment, since the wire guide 34E has the inclined wall 35C, the basket wire 41 can be easily inserted, and the opening and closing force of the basket 4 is reduced. In addition, a larger force is transmitted from the operation part 5 to the stone S. Furthermore, when the exposed position of the basket wire 41 is on the outer side in the radial direction R, the basket wire 41 can more easily follow the stone S when holding it. Therefore, a length of the tapered portion 32 in the longitudinal direction A can be shortened. As a result, the endoscope insertion performance is improved, and stability when the distal end surface 33 collides with the stone S is also improved.

The fourth embodiment of the present invention has been described above in detail with reference to the drawings, but the specific configuration is not limited to the embodiment, and design changes and the like are also included within the scope that does not deviate from the gist of the present invention. Furthermore, the components shown in the above-described embodiment and modified example can be appropriately combined to form a configuration.

In the above embodiments, the wire guides 34, 34C, 34D, and 34E are grooves, but the form of the wire guides is not limited thereto. The wire guide is not limited to a groove, but may be a guide lumen, at least a part of which is formed in a tubular shape.

### (Fifth embodiment)

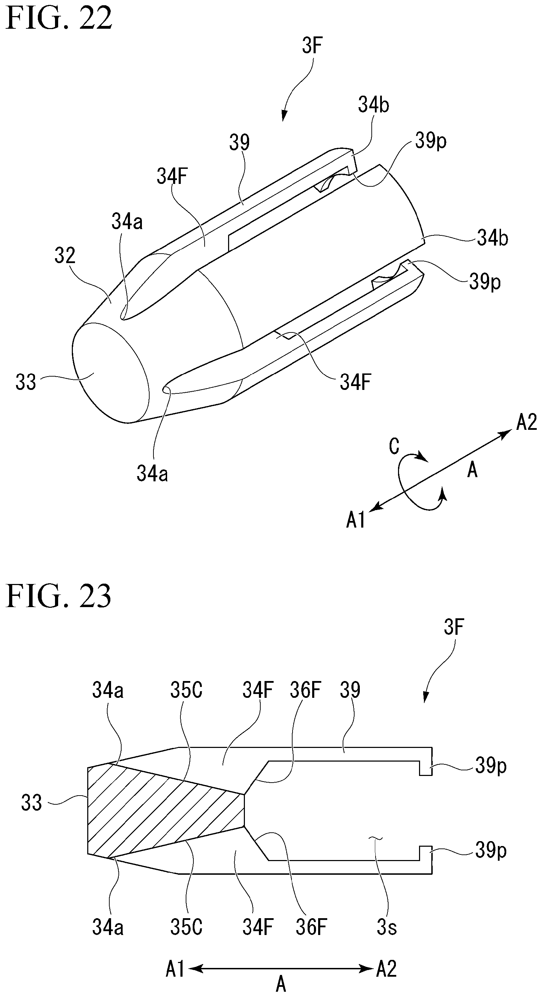

An attachment 3F according to a fifth embodiment of the present invention will be described with reference to FIGS. 22 to 25. In the following description, components common to those already described will be designated by the same reference numerals, and redundant description will be omitted.

FIG. 22 is a perspective view of the attachment 3F.

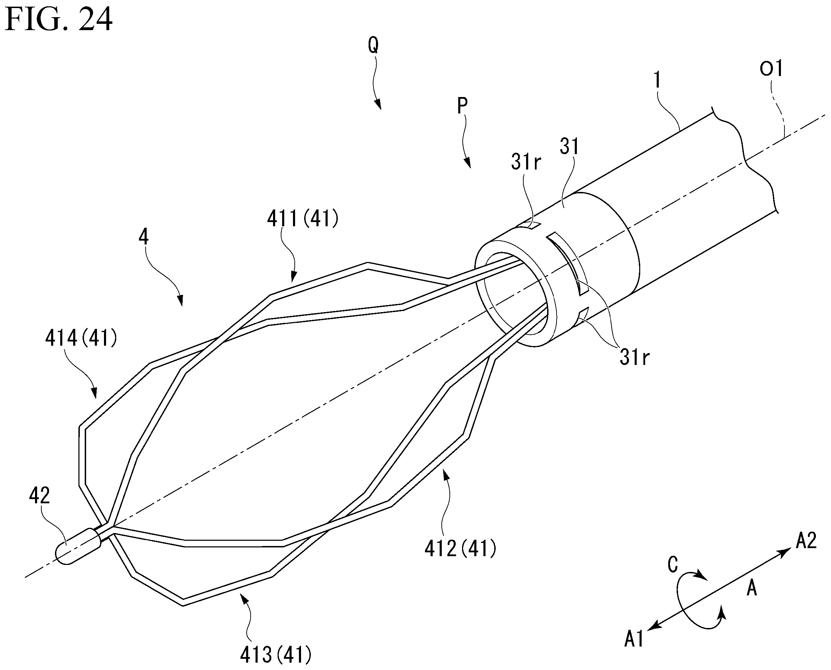

The attachment 3F is a component that can be attached to a conventional crushing tool Q as shown in FIG. 24. The attachment 3F is made of a metal such as stainless steel, and has a tapered portion 32 and an attachable and detachable portion 39.

The attachable and detachable portion 39 is formed in a cylindrical shape, and four wire guides 34F are formed in the circumferential direction C relative to the longitudinal direction A. A plurality of engaging projections 39p that protrude inward in the radial direction R are provided at an end portion of the attachable and detachable portion 39 on the proximal end side A1.

The wire guides 34F are grooves that extend in the longitudinal direction A on the outer circumference of the attachable and detachable portion 39. The four wire guides 34F are disposed at different positions in the circumferential direction C of the attachable and detachable portion 39. The four wire guides 34F are disposed outward in the radial direction R with respect to the central axis O3.

A distal end 34a of the wire guide 34F is located on a side surface 32s of the tapered portion 32. Therefore, the wire guide 34F does not extend to the distal end surface 33. In other words, the distal end surface 33 is located on the distal side A1 with respect to the distal end 34a of the wire guide 34F.

A proximal end 34b of the wire guide 34F extends to a proximal end of the attachable and detachable portion 39. In other words, the wire guide 34F is a slit.

FIG. 23 is a cross-sectional view of the attachment 3F.

The wire guide 34F has a guide lumen 36F that communicates with the internal space 3s of the attachable and detachable portion 39. The guide lumen 36F has an inclined surface 35C that inclines outward in the radial direction R from the proximal end to the distal end.

The number of basket wires 41 is not limited to four, and may be, for example, three, five, or six. The number of wire guides 34F is changed according to the number of basket wires 41.

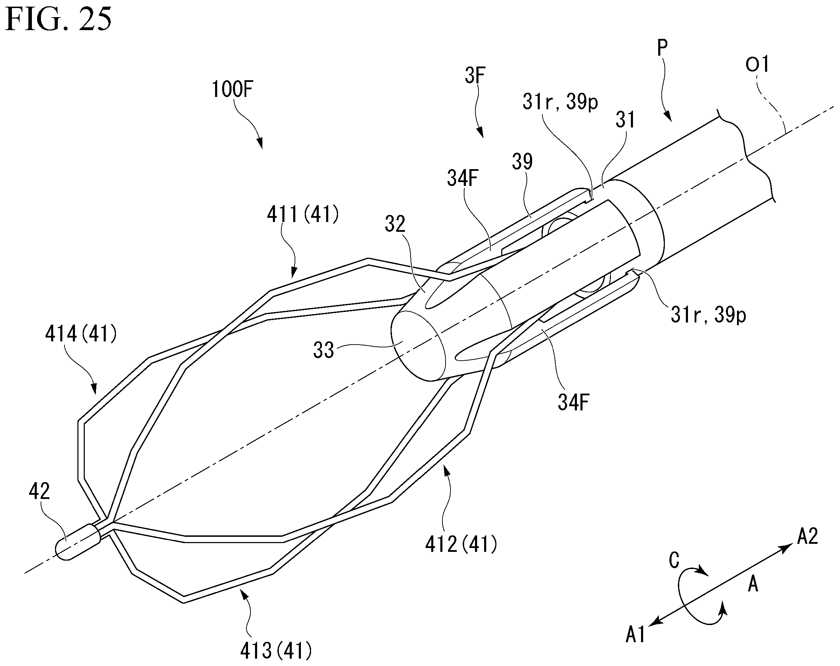

FIG. 25 is a perspective view of the attachment 3F attached to the distal end member P of the conventional crushing tool Q. The attachable and detachable portion 39 is attachable to and detachable from the distal end member P of the conventional crushing tool Q by fitting (including snap fitting). For example, as shown in FIG. 24, the main body 31 of the distal end member P of the crushing tool Q is provided with a plurality of engaging recesses 31r. As shown in FIG. 25, the attachable and detachable portion 39 is attached to the distal end member P by coupling the engaging projections 39p of the attachable and detachable portion 39 to the engaging recesses 31r of the distal end member P of the crushing tool Q by snap fitting. A method of attaching the attachable and detachable portion 39 is not limited to fitting. For example, the attachable and detachable portion 39 may be fixed to the distal end member P by an adhesive tape.

When the attachable and detachable portion 39 is attached to the distal end member P, the internal space 3s of the attachable and detachable portion 39 communicates with the internal space 1s of the sheath 1. In the state in which the attachable and detachable portion 39 is attached to the distal end member P, the distal end surface 33 is located closer to the distal tip 42 side (the distal side A1) than the distal end 34a of the wire guide 34F. Furthermore, in the state in which the attachable and detachable portion 39 is attached to the distal end member P, the basket wires 41 are inserted through the slit of the wire guide 34F, and when the basket wires 41 are advanced and retracted, the basket wires 41 are guided by the inclined surface 35C.

According to the attachment 3F of the embodiment, by attaching the attachment 3F to the conventional crushing tool Q, the conventional crushing tool Q can be used as a crushing tool 100F having the same functions as the crushing tool 100 of the first embodiment, or the like.

The fifth embodiment of the present invention has been described above in detail with reference to the drawings, but the specific configuration is not limited to the embodiment, and design changes and the like are also included within the scope that does not deviate from the gist of the present invention. Furthermore, the components shown in the above-described embodiment and modified example can be appropriately combined to form a configuration.

### INDUSTRIAL APPLICABILITY

The present invention can be applied to an endoscopic treatment tool having a basket wire.

### REFERENCE SIGNS LIST

300 Endoscopic treatment system
200 Endoscope
100, 100A, 100B, 100C, 100D, 100E, 100F Crushing tool (Endoscopic treatment tool)
1 Sheath
1s Internal space (lumen)
2 Operation wire
3, 3A, 3B, 3C Distal end member
3F Attachment
3s Internal space
31, 31C Main body
32 Tapered portion
32B Distal end portion
33, 33B Distal end surface
33h Opening
34, 34C, 34F Wire guide
341 First wire guide
342 Second wire guide
343 Third wire guide
344 Fourth wire guide
35 Wall
35C Inclined wall (inclined surface)
36 Opening (wire insertion part)
37 Protrusion
38 Slit
39 Attachable and detachable portion
4 Basket
41 Basket wire
411 First basket wire
412 Second basket wire
413 Third basket wire
414 Fourth basket wire
42 Distal tip
43 Bundled portion
5 Operation part
51 Operation part main body
52 Liquid supply port
53 Slider
54 Guide wire port
A Longitudinal direction
A1 Distal end side (distal side)
A2 Proximal end side (proximal side)
B Bile duct
C Circumferential direction
R Radial direction
S Stone

## Claims

1. A crushing tool comprising:
a sheath;
a distal end member attached to a distal end of the sheath; and
a basket wire configured to protrude from the distal end member,
wherein the distal end member has a wire guide that guides the basket wire, and
the wire guide is disposed outward in a radial direction with respect to a distal end surface of the distal end member.

2. The crushing tool according to claim 1, wherein a plurality of the wire guides are disposed at different positions in a circumferential direction of the distal end member.

3. The crushing tool according to claim 1, wherein the wire guide is separated by a wall provided inward of the distal end member in the radial direction.

4. The crushing tool according to claim 1, wherein the wire guide has an inclined surface that inclines outward from a proximal end toward a distal end of the distal end member in the radial direction.

5. The crushing tool according to claim 1, wherein the distal end member has a tapered portion of which a diameter decreases toward a distal end of the distal end member, and
a distal end of the wire guide is provided on the tapered portion.

6. The crushing tool according to claim 1, wherein the distal end surface of the distal end member is flat.

7. The crushing tool according to claim 1, wherein the distal end surface of the distal end member is a curved surface that is convex from a proximal end toward a distal end of the distal end member.

8. The crushing tool according to claim 1, wherein the distal end surface of the distal end member has at least three or more protrusions, and
the protrusions and distal ends of the wire guides are arranged alternately in a circumferential direction of the distal end member.

9. The crushing tool according to claim 1, wherein an opening is formed in the distal end surface of the distal end member, and
the opening is disposed inward in the radial direction with respect to a distal end of the wire guide.

10. The crushing tool according to claim 1, wherein an opening is formed in the distal end surface of the distal end member, and
the distal end surface of the distal end member is located on a distal side with respect to the opening.

11. The crushing tool according to claim 1, wherein the distal end surface of the distal end member has a slit that passes through a central axis of the distal end member and extends in the radial direction, and
the slit and distal ends of the wire guides are arranged alternately in a circumferential direction of the distal end member.

12. The crushing tool according to claim 1, wherein the distal end surface of the distal end member has a slit that passes through a central axis of the distal end member and extends in the radial direction, and
the slit is connected to a distal end of the wire guide.

13. The crushing tool according to claim 1, wherein the wire guide has a guide lumen.

14. The crushing tool according to claim 9, wherein the distal end member has a cylindrical main body and a tapered portion of which a diameter decreases toward a distal end of the distal end member, and
a length of the distal end surface of the distal end member in the radial direction is smaller than an inner diameter of the main body.

15. The crushing tool according to claim 14, wherein a maximum distance from the opening to the distal end surface is greater than the inner diameter of the main body.

16. An attachment for a crushing tool, which has a guide lumen through which a basket wire passes,
wherein the guide lumen has an inclined surface that inclines outward in a radial direction from a proximal end to a distal end thereof.

17. A crushing tool comprising:
a sheath;
a distal end member attached to a distal end of the sheath; and
a basket formed by a basket wire protruding from a distal end of the distal end member,
wherein when the basket wire is further pulled toward a proximal end side of the sheath in a state in which a stone is captured by the basket, the distal end of the distal end member is pressed against the stone.
